# EUROPÄISCHE PATENTSCHRIFT

(11) **EP 1 457 186 B1**
(45) Veröffentlichungstag und Bekanntmachung des Hinweises auf die Patenterteilung: **27.06.2007**
(21) Anmeldenummer: 04005569.1
(22) Anmeldetag: 09.03.2004
(51) Int. Cl.: A61F 15/00, B65H 35/00, B65D 83/08

(54) **Spender für Verbandmittel**
Dispenser for bandage material
Distributeur de bandage

(30) Priorität: 10.03.2003 DE 20303763 U
(43) Veröffentlichungstag der Anmeldung: 15.09.2004
(73) Patentinhaber: Lohmann & Rauscher GmbH & Co. KG, 56567 Neuwied (DE)
(72) Erfinder: Koch, Reinhard, 53489 Sinzig (DE); Ockenfeld, Dieter, 56736 Kottenheim (DE)
(74) Vertreter: Leinweber & Zimmermann

(56) Entgegenhaltungen:
- DE-U- 8 033 734
- DE-U- 8 526 163
- FR-A- 686 485
- GB-A- 938 995
- GB-A- 1 586 719
- US-A- 4 453 634

## Beschreibung

Die Erfindung betrifft einen Spender für Verbandmittel, insbesondere wickelförmige Mullbinden, mit einem von einer vorzugsweise im wesentlichen kreiszylindermantelförmigen achsparallelen Mantelflächenanordnung und zwei an den axialen Enden der Mantelflächenanordnung angeordneten Stirnflächenelementen begrenzten und mindestens eine Entnahmeöffnung aufweisenden Aufnahmeraum für die das Verbandmittel.

Spender dieser Art sind beispielsweise aus der DE-U-81 25 693 bekannt. Der bekannte Spender besteht aus einem von einem der Stirnflächenelemente gebildeten Boden mit einer einstückig angeformten achsparallelen Seitenwandung und einem zu dem Boden parallelen, abnehmbaren Deckel, wobei an dem Deckel ein unter Bildung eines Entnahmeschlitzes und eines Fixierschlitzes in eine in der achsparallelen Seitenwandung gebildete Aussparung aufgenommener Wandungsabschnitt angeformt ist. Bei der bekannten Vorrichtung wird der Wickel in dem aus dem Boden und der Seitenwandung gebildeten Bodenteil eingelegt, das freie Ende des Wickels durch den Entnahmeschlitz nach außen gezogen und innerhalb der Aussparung derart zurückgeschlagen, daß nach dem Aufsetzen des Deckelteils der asymmetrisch in die Aussparung hineinragende Wandungsabschnitt das freie Ende des Packungsgutes in dem Fixierspalt festhält. Zum Entnehmen des Verbandmittels wird das freie Ende aus dem Fixierspalt herausgerissen und dann die gewünschte Länge bequem und widerstandsfrei aus dem Entnahmeschlitz herausgezogen.

In der DE 80 33 734 U und der GB-A-1 586 719 sind Spender beschreiben, deren Aufnahmeraum durch zwei scharnierartig miteinanderverbundene Packungsmulden gebildet ist. Bei diesen bekannten Spendern werden die Packungsmulden durch an den der scharnierartigen Verbindung gegenüberliegenden Rändem vorgesehene komplementär zueinander ausgeführte Verbindungselemente miteinander verbunden.

Wie der vorstehenden Erläuterung zu entnehmen ist, sind die bekannten Spender in der Regel nur zum einmaligen Gebrauch gedacht. Sobald das in dem Spender aufgenommene Verbandmittel verbraucht ist, wird im allgemeinen ein neuer Spender benötigt. Das ist mit einem hohen Kostenaufwand verbunden.

Angesichts der vorstehend beschriebenen Probleme im Stand der Technik liegt der Erfindung die Aufgabe zugrunde, einen Spender der eingangs beschriebenen Art bereitzustellen, welcher zur kostengünstigen Bereitstellung von Verbandmitteln geeignet ist.

Erfindungsgemäß wird diese Aufgabe durch die in kennzeichnenden Teil des Patentanspruchs 1 angegebene Weiterbildung der bekannten Spender gelöst. Vorteilhafte ausführungstromen der Erfindung sind in den abhängigen Patentansprüchen angegeben Weiterbildung der bekannten Spender gelöst.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine Darstellung einer ersten Ausführungsform erfindungsgemäßer Spender,
- **Fig. 2**: eine Darstellung einer zweiten Ausführungsform erfindungsgemäßer Spender und
- **Fig. 3**: eine Darstellung einer dritten Ausführungsform erfindungsgemäßer Spender.

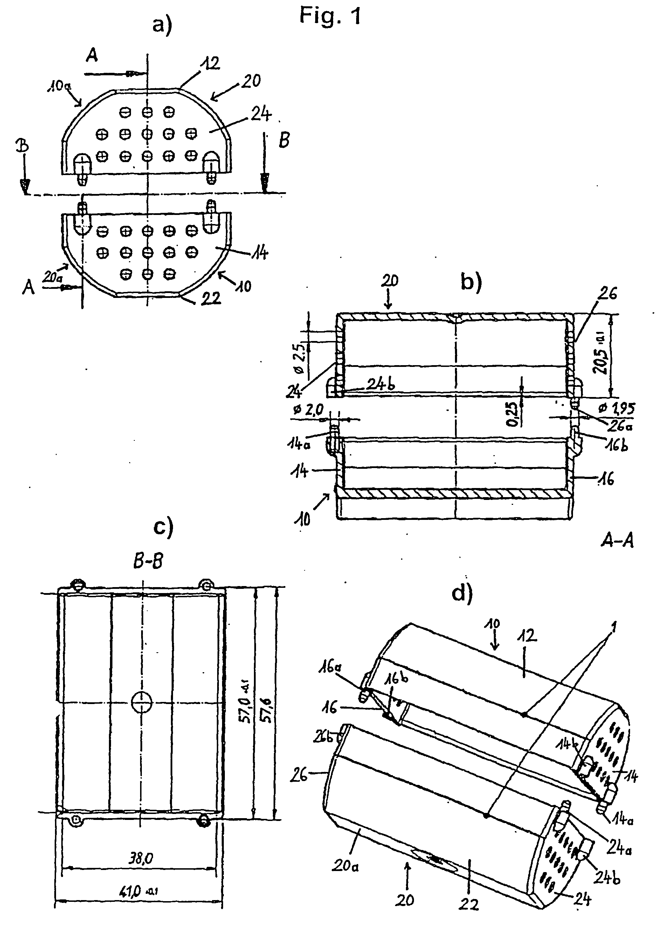

Fig. 1a) zeigt eine Ansicht eines erfindungsgemäßen Spenders, Fig. 1b) zeigt eine Schnittdarstellung längs der Schnittlinie A-A in Fig. 1a), Fig. 1c) zeigt eine Schnittdarstellung längs der Schnittlinie B-B in Fig. 1a) und Fig. 1d) zeigt eine perspektivische Darstellung des Spenders.

Der in Fig. 1 dargestellte Spender umfaßt zwei Bauelemente 10 und 20, von denen jedes ein Mantelflächensegment 12 bzw. 22 umfaßt. Jedes der Mantelflächensegmente 12 und 22 ist im wesentlichen in Form eines durch Schneiden eines Kreiszylindermantels längs einer axialen Schnittlinie gebildeten Kreiszylindermantelflächensegmentes gebildet. An beiden axialen Enden der Mantelflächensegmente 12 und 22 sind Stirnflächensegmente 14, 16, 24 und 26 angeordnet. Dabei können die Mantelflächensegmente 12 und 22 und die Stirnflächensegmente 14, 16, 24 und 26 einstückig durch Spritzgießen eines geeigneten Kunststoffes hergestellt werden. Die Mantelflächensegmente 12 und 22 und die Stirnflächensegmente 14, 16, 24 und 26 der einzelnen Bauteile 10 und 20 bilden einen dem jeweils anderen Bauteil gegenüberliegenden rechteckförmigen Rand. Jedes der Bauteile weist an zwei einander diagonal gegenüberliegenden Ecken des Randes einen Befestigungszapfen 14a, 16a bzw. 24a und 26a auf. An den beiden andern Ecken des rechteckförmigen Randes sind Aufnahmeöffnungen vorgesehen, in die ein Aufnahmebereich der Befestigungszapfen 14a, 16a, 24a bzw. 26a des jeweils gegenüberliegenden Aufnahmezapfens einführbar ist. Jeder der Befestigungszapfen 14a, 16a, 24a und 26a weist, ausgehend von dem Stirnflächensegment, zunächst einen Bereich großen Durchmessers auf, an den in axialer Richtung der Aufnahmebereich geringeren Durchmessers angrenzt. Dabei ist der Bereich größeren Durchmessers ein Spaltbildungsbereich, welcher nicht in die gegenüberliegende Aufnahmeöffnung einführbar ist, so daß nach Einführen der Aufnahmebereiche der Befestigungszapfen 14a, 16a, 24a und 26a in die jeweiligen Aufnahmeöffnungen 14b, 16b, 24b und 26b zwischen den einander gegenüberliegenden Rändern der Bauteile ein Spalt frei bleibt, welcher eine Entnahmeöffnung für das in dem Spender aufgenommene Verbandmittel bildet.

Jedes Bauteil weist im Bereich seines dem benachbarten Bauteil zugewandten Rand abgewandten Scheitels eine Abflachung 10a bzw. 20a auf, welche eine stabile Lagerung des Spenders erlaubt. Ferner sind im Bereich der Stirnflächensegmente 14, 16, 24 und 26 Durchbrechungen vorgesehen, welche eine Dampfdurchlässigkeit des Spenders während des Sterilisierens im Autoklaven gewährleisten.

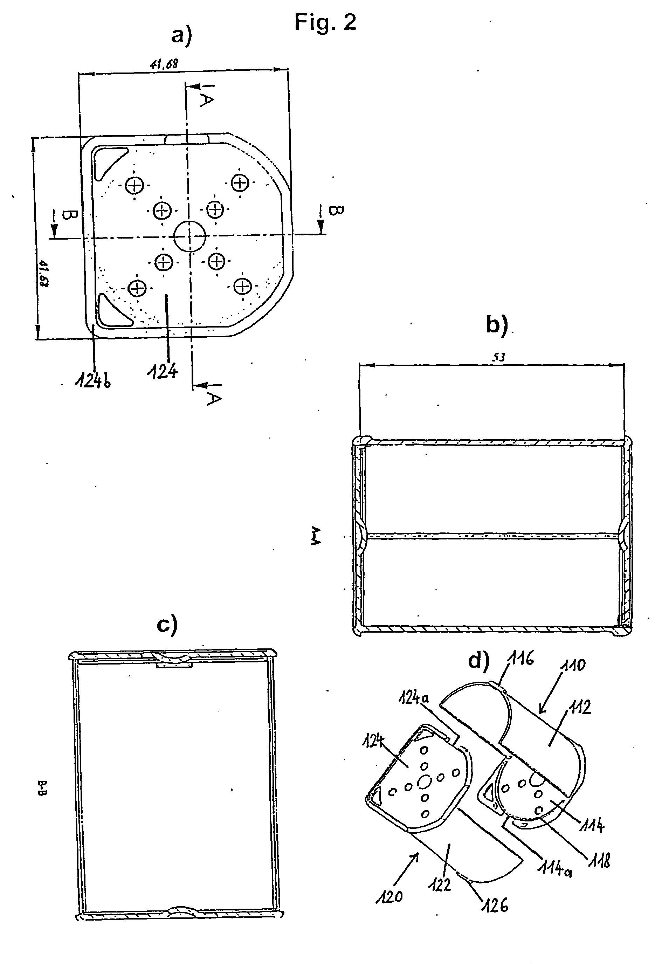

Fig. 2a) zeigt eine Ansicht eines Spenders gemäß einer zweiten Ausführungsform der Erfindung. Fig. 2b) zeigt eine Schnittdarstellung des in Fig. 2a) dargestellten Spenders längs der Schnittlinie A-A. Fig. 2c) zeigt eine Schnittdarstellung des in Fig. 2a) dargestellten Spenders längs der Schnittebene B-B und Fig. 2d) zeigt eine explosionsartige perspektivische Darstellung des in Fig. 2a) dargestellten Spenders.

Wie besonders deutlich in Fig. 2d) dargestellt ist, weist der in Fig. 2 dargestellte Spender zwei formgleiche Bauteile auf, von denen jeder ein Mantelflächensegment 112 bzw. 122 und ein an einem axialen Ende des Mantelflächensegmentes 112 bzw. 122 angeordnetes Stirnflächensegment 114 bzw. 124 umfaßt. Dabei sind die Mantelflächensegmente 112 und 122 auch bei der in Fig. 2 dargestellten Ausführungsform der Erfindung im wesentlichen in Form von Kreiszylindermantelsegmenten gebildet, die durch Schneiden eines Kreiszylindermantels längs einer axialen Schnittebene erhalten werden. Auch sind die Stirnflächensegmente 114 und 124 im wesentlichen eben und senkrecht zur Zylinderachse verlaufend angeordnet. Jedes der Mantelflächensegmente 112 und 122 weist an seinem dem entsprechenden Stirnflächensegment 114 bzw. 124 abgewandten Rand ein Befestigungselement 116 bzw. 126 auf, welches im montierten Zustand eine Ausnehmung 124a bzw. 114a in dem Rand des anderen Bauteils 120 bzw. 110 durchgreift und dieses Stirnflächenelement übergreift. Ferner weisen die Stirnflächensegmente 114 und 116 jeweils ein sich in den Innenraum des Spenders erstreckendes Anlageelement 118 bzw. 128 auf, an dem eine innere Begrenzungsfläche des Mantelflächensegmentes 122 bzw. 112 des jeweils anderen Bauteils im montierten Zustand anliegt, um so eine große Stabilität des Spenders bereitzustellen.

Wie besonders deutlich in Fig. 2b) dargestellt, weisen die Stirnflächensegmente 114 und 124 einen etwa geradlinig verlaufenden freiliegenden Randabschnitt 124b bzw. 114b auf. Auch bei der in Fig. 2 dargestellten Ausführungsform der Erfindung sind im Bereich der Stirnflächensegmente 114 und 124 Durchbrechungen vorgesehen, welche die Sterilisationsfähigkeit des Spenders gewährleisten.

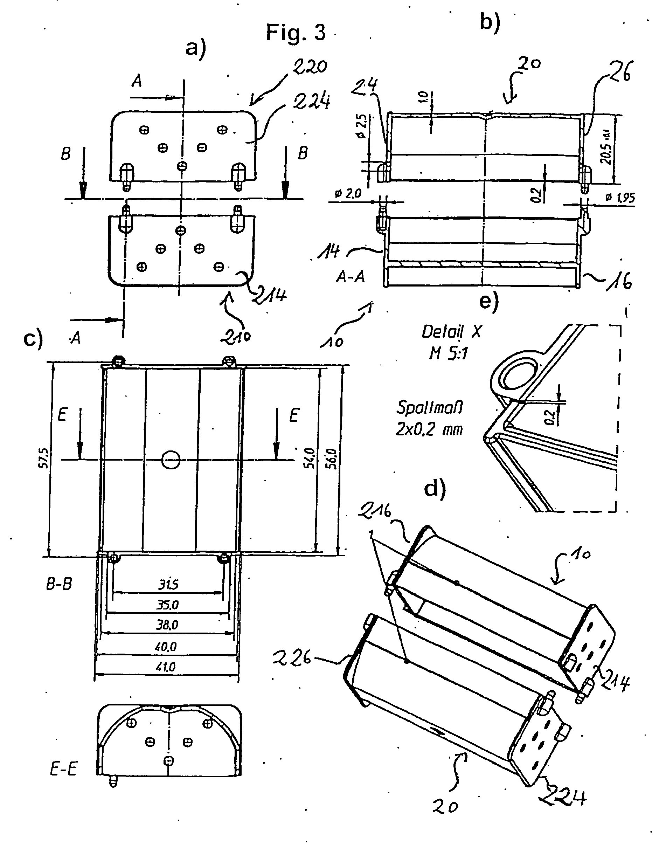

Die in Fig. 3 dargestellte Ausführungsform der Erfindung unterscheidet sich im wesentlichen nur dadurch von der anhand der Fig. 1 erläuterten Ausführungsform, daß die seitlichen Ränder der Stirnflächensegmente 214, 216, 224 und 226 über das Mantelflächensegment hinaus verlängert sind. Dadurch wird eine dem Kippen des Spenders entgegenwirkende Stabilisierung erreicht. Bei der in Fig. 3 dargestellten Ausführungsform der Erfindung hat das Mantelflächensegment des Spenders keine Bodenberührung, wie besonders deutlich anhand der Fig. 3 b) zu erkennen ist. Die Stirnflächensegmente 214, 216, 224 und 226 bilden daher als zusätzliche Füße die Standfläche des Spenders. Auf diese Weise wird eine weitere Stabilisierung erreicht.

Wie besonders deutlich in Fig. 3 e) zu erkennen ist, wird bei der in dieser Figur dargestellten Ausführungsform der Erfindung die durch das Zusammenfügen entstehende Spenderöffnung seitlich bis zum jeweiligen Befestigungszapfen weitergeführt. Auf diese Weise wird einem Verhaken des Verbandmitttels beim Montieren des Spenders entgegengewirkt. Schließlich ist noch darauf hinzuweisen, daß die Löcher in den Stirnflächensegmenten der Spendergeometrie angepaßt wurden.

Die Erfindung ist nicht auf die anhand der Zeichnung erläuterten Ausführungsbeispiele beschränkt. Vielmehr ist auch an die Bereitstellung von Spendern gedacht, bei denen die Mantelflächensegmente polygonale Flächen bilden. Ferner können die einzelnen Bauteile erfindungsgemäßer Spender auch dauerhaft miteinander verbunden werden, etwa durch eine Verklebung der einzelnen Bauteile. Auch ist daran gedacht, im Bereich der einzelnen Mantelflächensegmente zwei oder mehr Entnahmeöffnungen bereitzustellen.

## Patentansprüche

1. Spender für Verbandmittel, insbesondere Mullbinden, mit einem von einer achsparallelen Mantelflächenanordnung und zwei an den axialen Enden der Mantelflächenanordnung angeordneten Stirnflächenelementen begrenzten und mindestens eine Entnahmeöffnung aufweisenden Aufnahmeraum für die Verbandmittel, **dadurch gekennzeichnet, daß** die Mantelflächenanordnung und die Stirnflächenelemente aus insgesamt zwei formgleichen Bauteilen gebildet sind.

2. Spender nach Anspruch 1, **dadurch gekennzeichnet, daß** jedes Bauteil ein sich über die gesamte axiale Länge der Mantelflächenanordnung erstreckendes Mantelflächensegment aufweist.

3. Spender nach Anspruch 1 oder 2, **dadurch gekennzeichnet, daß** jedes Bauteil zwei an den axialen Enden des Mantelflächensegmentes angeordnete Stirnflächensegmente aufweist.

4. Spender nach den Ansprüchen 2 und 3, **dadurch gekennzeichnet, daß** das Mantelflächensegment und die Stirnflächensegmente einen im montierten Zustand dem jeweils anderen Bauteil gegenüberliegenden, etwa rechteckförmigen Bauteilrand bilden.

5. Spender nach Anspruch 4, **dadurch gekennzeichnet, daß** an mindestens einer Ecke, insbesondere an zwei diagonal gegenüberliegenden Ecken des Bauteilrandes, ein Befestigungszapfen und an mindestens einer Ecke, insbesondere zwei diagonal gegenüberliegenden Ecken des Bauteilrandes, eine zum Aufnehmen eines Aufnahmebereichs eines Befestigungszapfens ausgelegte Aufnahmeöffnung angeordnet ist.

6. Spender nach Anspruch 5, **dadurch gekennzeichnet, daß** mindestens ein Befestigungszapfen ausgehend von dem Bauteilrand zunächst einen nicht in die Aufnahmeöffnung des im montierten Zustand gegenüberliegenden Bauteils einführbaren Spaltbildungsbereich und in axialer Richtung daran angrenzend den in die Aufnahmeöffnung einführbaren Aufnahmebereich aufweist.

7. Spender nach Anspruch 1 oder 2, **dadurch gekennzeichnet, daß** jedes Bauteil an einem axialen Ende des Mantelflächensegmentes ein eine vollständige Stirnfläche des Aufnahmeraumes bildendes Stirnflächensegment aufweist.

8. Spender nach Anspruch 7, **dadurch gekennzeichnet, daß** an dem dem Stirnflächensegment abgewandten Rand des Mantelflächensegmentes ein zum Festlegen des Bauteils an dem Stirnflächensegment des anderen Bauteils ausgelegtes Befestigungselement angeordnet ist.

9. Spender nach Anspruch 8, **dadurch gekennzeichnet, daß** das Befestigungselement das Stirnflächensegment des anderen Bauteils im montierten Zustand übergreift.

10. Spender nach einem der Ansprüche 7 - 9, **dadurch gekennzeichnet, daß** das Stirnflächensegment ein in den Aufnahmeraum ragendes Anlageelement aufweist, an dem eine innere Begrenzungsfläche des Mantelflächensegmentes des anderen Bauteils im montierten Zustand anliegt.

11. Spender nach einem der vorhergehenden Ansprüche, **dadurch gekennzeichnet, daß** mindestens ein Stirnflächenelement einen freiliegenden, etwa geradlinig verlaufenden Randabschnitt aufweist.

12. Spender nach einem der vorhergehenden Ansprüche, **dadurch gekennzeichnet, daß** die Mantelflächenanordnung und/oder mindestens ein Stirnflächenelement mindestens eine Durchbrechung aufweist.

13. Spender nach einem der vorhergehenden Ansprüche, **dadurch gekennzeichnet, daß** die Mantelflächenanordnung kreiszylindermantelförmig ist.

## Claims

1. A dispenser for binding means, in particular gauze bandages, with a space for retaining the binding means defined by a shell surface arrangement parallel to the axis and two face surface elements disposed at the axial ends of the shell surface arrangement and having at least one extraction opening, **characterised in that** the shell surface arrangement and the face surface elements are formed by a total of two components identical in form.

2. The dispenser according to Claim 1, **characterised in that** each component has a shell surface segment extending over the whole axial length of the shell surface arrangement.

3. The dispenser according to Claim 1 or 2, **characterised in that** each component has two face surface segments disposed on the axial ends of the shell surface segment.

4. The dispenser according to Claims 2 and 3, **characterised in that** in the assembled state, the shell surface segment and the face surface segments form an approximately rectangular component edge lying opposite the respective other component.

5. The dispenser according to Claim 4, **characterised in that** disposed at at least one corner, and in particular at two diagonally opposite corners of the component edge, there is an attachment pin, and at at least one corner, and in particular at two diagonally opposite corners of the component edge, there is a retainer opening designed to retain a retaining region of an attachment pin.

6. The dispenser according to Claim 5, **characterised in that** at least one attachment pin has a gap formation region starting from the component edge which can first of all not be introduced into the retainer opening of the component lying opposite in the assembled state and in the axial direction adjacent to this the retaining region which can be introduced into the retainer opening.

7. The dispenser according to Claim 1 or 2, **characterised in that** each component has at one axial end of the shell surface segment a face surface segment forming a complete face surface of the retaining space.

8. The dispenser according to Claim 7, **characterised in that** an attachment element designed to fix the component to the face surface segment of the other component is disposed on the edge of the shell surface segment facing away from the face surface segment.

9. The dispenser according to Claim 8, **characterised in that** the attachment element overlaps the face surface segment of the other component in the assembled state.

10. The dispenser according to any of Claims 7 - 9, **characterised in that** the face surface segment has a support element projecting into the retaining space on which an inner limit surface of the shell surface segment of the other component rests in the assembled state.

11. The dispenser according to any of the preceding claims, **characterised in that** at least one face surface element has an open edge section with extends in an approximately straight line.

12. The dispenser according to any of the preceding claims, **characterised in that** the shell surface arrangement and/or at least one face surface element has at least one through hole.

13. The dispenser according to any of the preceding claims, **characterised in that** the shell surface arrangement is in the form of a circular cylinder shell.

## Revendications

1. Distributeur de bandage, en particulier de bandage en mousseline, présentant pour le bandage un volume d'accueil, délimité par un système d'enveloppes à axes parallèles et par deux éléments de face frontale montés aux extrémités axiales du système d'enveloppes, ainsi qu'au moins une ouverture d'extraction, **caractérisé en ce que** l'ensemble du système d'enveloppe et des éléments de face frontale est constitué par deux composants de même forme.

2. Distributeur selon la revendication 1, **caractérisé en ce que** chaque composant présente un segment d'enveloppe s'étendant sur toute la longueur axiale du système d'enveloppes.

3. Distributeur selon la revendication 1 ou 2, **caractérisé en ce que** chaque composant présente des segments de face frontale disposés aux extrémités axiales du segment d'enveloppe.

4. Distributeur selon les revendications 2 et 3, **caractérisé en ce que** le segment d'enveloppe et les segments de face frontale correspondants présentent à l'état monté, un bord à peu près rectangulaire faisant face à l'autre composant.

5. Distributeur selon la revendication 4, **caractérisé en ce qu'**il est prévu sur au moins un angle, et en particulier sur deux angles diagonalement opposés du bord du composant, un téton de fixation, et sur au moins un angle, et en particulier sur deux angles diagonalement opposés du bord du composant, une ouverture d'accueil conçue pour recevoir une zone d'accueil d'un téton de fixation.

6. Distributeur selon la revendication 5, **caractérisé en ce qu'**au moins un téton de fixation, en partant du bord du composant, présente d'abord une zone de formation de fente qui ne peut pas être introduite dans l'ouverture d'accueil du composant situé à l'opposé à l'état monté, puis, en direction axiale, présente en limite de la zone précédente la zone d'accueil qui peut être introduite dans l'ouverture d'accueil.

7. Distributeur selon la revendication 1 ou 2, **caractérisé en ce que** chaque composant présente sur une extrémité axiale du segment d'enveloppe, un segment de face frontale constituant la face frontale totale du volume d'accueil.

8. Distributeur selon la revendication 7, **caractérisé en ce que** sur le bord du segment d'enveloppe éloigné du segment de face frontale correspondant, est disposé un élément de fixation conçu pour fixer le composant sur le segment de face frontale de l'autre composant.

9. Distributeur selon la revendication 8, **caractérisé en ce que** l'élément de fixation est, à l'état monté, en prise par dessus avec le segment de face frontale de l'autre composant.

10. Distributeur selon une des revendications 7 à 9, **caractérisé en ce que** le segment de face frontale présente un élément d'appui faisant saillie dans le volume d'accueil et sur lequel, à l'état monté, est appliquée la surface interne en limite du segment d'enveloppe de l'autre composant.

11. Distributeur selon une des revendications précédentes, **caractérisé en ce qu'**au moins un élément de face frontale présente une zone marginale libre, à peu près rectiligne.

12. Distributeur selon une des revendications précédentes, **caractérisé en ce que** le système d'enveloppes et/ou au moins un élément de face frontale présente au moins un passage.

13. Distributeur selon une des revendications précédentes, **caractérisé en ce que** le système d'enveloppes a la forme d'un cylindre circulaire.
